Europäisches Patentamt

(19) European Patent Office

Office européen des brevets

(11) Publication number: **0 213 685**
**B1**

(12) EUROPEAN PATENT SPECIFICATION

(45) Date of publication of patent specification: **25.07.90**

(51) Int. Cl.⁵: **C 07 D 499/42**

(21) Application number: **86201510.4**

(22) Date of filing: **02.09.86**

(54) **Process for the preparation of 6-amino penicillanic acid 1,1 dioxide by oxidation of 6-amino penicillanic acid.**

(30) Priority: **06.09.85 EP 85201409**

(43) Date of publication of application:
**11.03.87 Bulletin 87/11**

(45) Publication of the grant of the patent:
**25.07.90 Bulletin 90/30**

(84) Designated Contracting States:
**AT BE CH DE FR GB IT LI LU NL SE**

(56) References cited:
**EP-A-0 109 818**

**CHEMICAL ABSTRACTS, vol. 97, no. 1, 05 July 1982, Columbus, OH (US); p. 586, no. 6064y**

(73) Proprietor: **GIST-BROCADES N.V.**
**Wateringseweg 1**
**NL-2611 XT Delft (NL)**

(72) Inventor: **Bos, Jacobus Johannes**
**Landluststraat 88**
**NL-2804 KT Gouda (NL)**
Inventor: **Cuperus, Rinze**
**Julianalaan 11**
**NL-2641 HA Pijnacker (NL)**
Inventor: **Wielinga, Rudolf**
**Meidoornlaan 58**
**NL-2678 RG De Lier (NL)**

(74) Representative: **Huygens, Arthur Victor, Dr. et al**
**c/o Gist-Brocades N.V. Patent & Trademarks Department Wateringseweg 1 PO Box 1**
**NL-2600 MA Delft (NL)**

Courier Press, Leamington Spa, England.

**Description**

The invention relates to a new process for the preparation of 6-amino penicillanic acid 1,1-dioxide by direct oxidation of 6-amino penicillanic acid or 6-amino penicillanic acid sulfoxide.

One of today's most well-known and widely used classes of antibacterial compounds are the beta-lactam antibiotics. These have a 2-azetidinone (beta-lactam) ring fused to a thiazolidine ring (penicillins) or a dihydro-1,3-thiazine ring (cephalosporins). Typical examples of penicillins are benzylpenicillin (penicillin G), phenoxymethylpenicillin (penicillin V), ampicillin and amoxycillin. However, certain penicillins are inactive or almost inactive against certain microorganisms, due, it is thought, to the production of a beta-lactamase by the microorganism.

Beta-lactamases are enzymes which cleave the beta-lactam ring of the penicillin leading to decomposition products which do not possess antibacterial activity. This is a growing problem in that more bacteria become resistant to penicillins by the acquisition of the ability to produce beta-lactamases. However, several new classes of compounds have been discovered which inhibit beta-lactamases and, when used in combination with a penicillin, can increase or enhance the antibacterial activity of the penicillin against the bacteria.

EP—A—0002927 describes the use of 6-amino penicillanic acid 1,1-dioxide and its salts to enhance the antibacterial activity of beta-lactam antibiotic. NL—A—7806126 discloses that penicillanic acid 1,1-dioxide and its salts and esters have useful pharmacological properties, for example as effective inhibitors of several types of beta-lactamases present in various bacteria. Penicillanic acid 1,1-dioxide can be prepared from 6-amino penicillanic acid 1,1-dioxide by diazotising the 6-amino penicillanic acid and subsequently brominating the diazotised compounds, followed by the debromination of the brominated products, as described in EP—A—0093465 and 0092286. It will be appreciated therefore, that 6-amino penicillanic acid 1,1-dioxide is a valuable compound.

In EP—A—0109818 the oxidation of N-protected penicillins preparing the corresponding penicillin sulfones using potassium hydrogen persulfate is described. In JP—A—8216889 in a comparable process potassium permanganate is used.

EP—A—0002927 describes the preparation of 6-amino penicillanic acid 1,1-dioxide by oxidation of a 6-amino penicillanic acid derivative in which the 6-amino group and preferably also the 3-carboxyl group have been protected, using an oxidising agent such as potassium permanganate or 3-chloro-perbenzoic acid, followed by removing the protecting groups. This method has the disadvantage therefore, that the 6-amino group and usually also the 3-carboxylic acid group must be protected with protecting groups which must be removed after the oxidation without affecting the ring structure or bringing about other undesired structure changes in the molecule.

The direct oxidation of 6-amino-penicillanic acid into 6-amino-penicillanic acid 1,1-dioxide has not been described in the literature. Previous proposals for oxidation of 6-amino penicillanic acid have always resulted in the formation of the corresponding sulfoxides. For instance, J. Org. Chem. *30*, 4388 (1965) describes the oxidation of 6-amino-penicillanic acid into its sulfoxide using sodium metaperiodate. A yield of only 8% was obtained. J. Org. Chem. *37*, 793 (1972) describes the conversion of 6-amino-penicillanic acid into its sulfoxide using ozone. A yield of 95% was reported. Although sulfoxides are generally oxidized to sulfones by an excess of ozone, the further oxidation of 6-amino penicillanic acid sulfoxide to the sulfone with a large excess of ozone did not occur under these conditions. The oxidation of 6-amino-penicillanic acid into its sulfoxide using peracetic acid has been described in Synthesis, 264 (1976). A yield of 49% was reported.

We have now surprisingly found that 6-amino-penicillanic acid can be directly oxidised to 6-amino penicillanic acid 1,1-dioxide without the need for protection of either the 6-amino group or the 3-carboxyl group during the oxidation if the oxidation is carried out with a permanganate, for instance potassium or sodium permanganate, in an aqueous medium.

By operating in accordance with the present invention, 6-amino penicillanic acid 1,1-dioxide can be prepared in a two steps synthesis from benzylpenicillin (penicillin G), which is the most important starting material for all semi-synthetic penicillin compounds. Benzylpenicillin is produced in large amounts by fermentation and can be converted into 6-amino penicillanic acid in yields over 90%.

In accordance with the present invention, 6-amino-penicillanic acid is dissolved in an aqueous solution, for instance a diluted aqueous solution of sulphuric acid, to which optionally a co-solvent can be added, for instance acetonitrile, followed by addition of an aqueous solution of the permanganate.

It is also possible to add a mixture of permanganate in water and aqueous sulphuric acid to a suspension of 6-amino penicillanic acid in water, or in a mixture of water and an inert organic solvent, for instance acetonitrile. In this case it is possible to conduct the oxidation under neutral or slightly alkaline conditions, for instance by adjusting the pH to 7 or 8 by addition of ammonia.

It is also possible to bring about the oxidation by adding solid 6-amino penicillanic acid to a mixture of the permanganate and sulphuric acid in water or a mixture of water and an inert water miscible organic solvent, for instance acetonitrile.

Any excess of permanganate remaining can be removed by methods known in the art, for instance by the addition of sodium-meta-bisulphite.

The 6-amino penicillanic acid 1,1-dioxide can be isolated from the reaction mixture by adjusting the pH at approximately 3.3, at which pH the product crystallizes from the reaction mixture.

According to the above described reaction it is possible to obtain conversion yields of 85% or more of 6-amino penicillanic acid into its 1,1-dioxide.

The oxidation reaction is carried out at a temperature between −10°C and 20°C, preferably between −10°C and 0°C.

Instead carrying out the oxidation in the presence of sulphuric acid, it is also possible to use other acids, for instance phosphoric acid.

Instead of 6-amino penicillanic acid as starting material it is also possible to use 6-amino penicillanic acid sulfoxide.

The following examples illustrate the invention, without the intention of limit the scope of the invention to these specific embodiments.

Example I

6-Amino penicillanic acid (25 g, 116 mmol) was suspended in a mixture of water (80 ml) and acetonitrile (120 ml). The reaction mixture was cooled to −10°C and a solution of potassium permanganate (24 g, 152 mmol) and concentrated sulphuric acid (15 ml) in water (300 ml), was added at this temperature in 20 minutes. Excess potassium permanganate was destroyed by addition of a concentrated solution of sodium-meta-bisulphite (50%). The pH of the solution was adjusted to 3.2 by addition of ammonia during which addition the 6-amino penicillanic acid 1,1-dioxide crystallized. The solid mass was filtered off, washed with water/acetone and dried. 24.4 g solid mass with a content of 6-amino penicillanic acid 1,1-dioxide of 89% as determined by HPLC, were isolated. The yield was therefore 76%. The mother liquid contained another 9%.

Example II

6-Amino penicillanic acid (25 g, 116 mmol) was suspended in a mixture of water (80 ml) and acetonitrile (120 ml). The reaction mixture was cooled to −8°C, after which the pH of the reaction mixture was adjusted to 8.1 by addition of ammonia. To the reaction mixture a solution of potassium permanganate (23.2 g, 147 mmol) and phosphoric acid (7 ml, 85%) in water (100 ml) was added in 35 minutes while maintaining the temperature at −8°C and the pH at 7.5 by addition of ammonia. After stirring for 30 minutes at −8°C and pH 7.5 the mixture was filtered, the pH was adjusted to 4.0 by adding 4N HCl solution, and 1 ml of saturated sodium-meta-bisulphite solution was added. The pH was lowered to 3.2 by a further addition of hydrochloric acid (4N) and the mixture stirred at −8°C for 15 minutes. The 6-amino penicillanic acid 1,1-dioxide crystallized under these conditions. The solid mass was filtered off, washed with water/acetone and dried. 22.5 g solid mass with a content of 6-amino penicillanic acid 1,1-dioxide of 92.5% as determined by HPLC, were isolated. The yield was therefore 73%.

Example III

Potassium permanganate (22.5 g, 142 mmol) was dissolved in a mixture of aqueous sulphuric acid (100 ml, 6N) and water (175 ml). After the potassium permanganate was dissolved, acetonitrile (200 ml) was added. To this solution solid 6-amino penicillanic acid (21.6 g, 100 mmol) was added at −5°C at such a rate that the temperature did not exceed 0°C, followed by stirring at −5°C—0°C. Sodium-meta-bisulphite (5 g, 26 mmol) was added to the solution, whereafter the pH was adjusted at 3.3 by the addition of ammonia. At this pH, 6-amino penicillanic acid 1,1-dioxide crystallized. The solid was filtered off and dried. 18.6 g solid mass with a content of 6-amino penicillanic acid 1,1-dioxide of 90% as determined by HPLC, were isolated. The yield was therefore 68%.

Example IV

6-Amino penicillanic acid (25 g, 116 mmol) was suspended in a mixture of water (80 ml) and acetonitrile (120 ml). The reaction mixture was cooled to −5°C, after which the pH of the mixture was adjusted to 7.0 by addition of ammonia. To the reaction mixture a solution of sodium permanganate (21.5 g, 136 mmol) and phosphoric acid (7.5 ml, 85%) in water (25 ml) was added in 60 minutes while maintaining the temperature at −5°C and the pH at 7.0 by addition of ammonia. After stirring for 30 minutes at −5°C and pH 7.0, the mixture was filtered off, the pH was adjusted to 4.0 by adding 6N $H_2SO_4$ solution, and 1 ml of saturated sodium-meta-bisulphite solution was added. The pH was lowered to 3.2 by a further addition of acid and the mixture stirred at −5°C for 15 minutes. The 6-amino penicillanic acid 1,1-dioxide crystallized under these conditions.

The crystals were filtered off, washed and dried. 19,0 g solid mass with a content of 6-amino penicillanic acid 1,1-dioxide of 91% as determined by HPLC, were isolated. The yield was therefore 60%.

Example V

In a series of experiments the reactions of 6-amino penicillanic acid with various oxidising agents were carried out to compare with the oxidation with permanganate under the conditions as described in the Examples I—IV. The results are summarized in the table.

| Oxidising agent | Conditions | HPLC | | |
|---|---|---|---|---|
| | | % 6-APA | % 6-APA Sulfoxide | % 6-APA 1,1-Dioxide |
| KHSO$_5$ | a | 3 | 46 | — |
| H$_2$O$_2$+ZrOCl$_2$ | a | 35 | 20 | — |
| m-Chloroperbenzoic acid | a | 23 | 44 | — |
| Trifluoroperacetic acid | a | 51 | 22 | — |
| Performic acid | a | — | 40 | — |
| Sodium perborate | a | 9 | 16 | — |
| Sodium dichromate | b | 80 | — | — |
| Sodium perborate | b | 29 | 50 | — |
| H$_2$O$_2$+ZrOCl$_2$ | b | 15 | 30 | — |
| H$_2$O$_2$+ZrCl$_4$ | b | 5 | 81 | — |
| H$_2$O$_2$+ZrOCl$_2$ | c | — | — | — |
| m-Chloroperbenzoic acid | d | — | 100 | — |
| m-Chloroperbenzoic acid/ trifluoroperacetic acid | d | — | — | — |
| Performic acid | d | 90 | 10 | — |

a 6-Amino penicillanic acid (10 g, 46 mmol) was suspended in water, water/acetone, water/acetonitrile or water/glacial acetic acid. The oxidising agent (90—150 mmol) was added and the mixture stirred at 10°C—20°C.

b 6-Amino penicillanic acid (10 g, 46 mmol) was solved in H$_2$O/H$_2$SO$_4$ at pH 1.0. The oxidising agent (130—150 mmol) was added and the mixture stirred at 10°C—20°C.

c 6-Amino penicillanic acid (10 g, 46 mmol) was solved in H$_2$O/ammonia or H$_2$O/triethylamine at pH 7. The oxidising agent (150 mmol) was added and the mixture stirred at 15°C.

d 6-Amino penicillanic acid (10 g, 46 mmol) was suspended in a mixture of H$_2$O/dichloromethane and the phase transfer catalyst tetrabutylammonium hydrogensulphate. The oxidising agent (92—130 mmol) was added and the mixture stirred at 10°C.

Example VI

A process was carired out as described in Example I with the only difference that the half of the solution containing potassium permanganate was used, i.e. potassium permanganate (12 g, 76 mmol) and concentrated sulphuric acid (7.5 ml) in water (150 ml) was added to the 6-amino penicillanic acid (25 g) solution. 19 g solid mass with a content of 6-amino penicillanic acid 1,1-dioxide of 49% and of unreacted 6-amino penicillanic acid of 45% as determined by HPLC, were isolated. The yield was therefore 32%.

Example VII

6-Amino penicillanic acid sulfoxide (1-oxide) was prepared from 6-amino penicillanic acid according to Synthesis, 264 (1976).

6-Amino penicillanic acid sulfoxide (26.8 g, 116 mmol) was suspended in a mixture of water (100 ml) and acetonitrile (100 ml) at room temperature. To the reaction mixture a solution of potassium permanganate (22.5 g, 142 mmol) and sulfuric acid (75 ml, 6N) in 250 ml water and 250 ml acetonitrile was added slowly while maintaining the temperature between −10°C and −5°C. Sodium-meta-bisulphite (100 ml, 15%) was added to the solution, whereafter the pH was adjusted at 3,5 by the addition of ammonia. The formed crystals were filtered off, washed with water and dried. 16.2 g solid mass with a content of 6-amino penicillanic acid 1,1-dioxide of 90.5% as determined by HPLC, were isolated. The yield was therefore 51%.

**Claims**

1. A process for the preparation of 6-amino penicillanic acid 1,1-dioxide, characterized in that the 6-amino penicillanic acid or 6-amino penicillanic acid sulfoxide is oxidized with a permanganate in an aqueous medium.

2. Process according to claim 1, characterized in that an excess of permanganate is used.

3. Process according to claims 1 and 2, characterized in that the permanganate is potassium or sodium permanganate.

4. Process according to claims 1, 2 and 3, characterized in that the reaction is carried out between −10°C and 20°C.

5. Process according to claim 4, characterized in that the reaction is carried out between −10°C and 0°C.

# EP 0 213 685 B1

**Patentansprüche**

1. Verfahren zur Herstellung von 6-Aminopenicillansäure-1,1-dioxid, dadurch gekennzeichnet, dass man 6-Aminopenicillansäure oder 6-Aminopenicillansäuresulfoxid mit einem Permanganat in einem wässrigen Medium oxidiert.

2. Verfahren nach Anspruch 1, dadurch gekennzeichnet, dass man einen Permanganatüberschuss anwendet.

3. Verfahren nach den Ansprüchen 1 und 2, dadurch gekennzeichnet, dass das Permanganat Kalium- oder Natriumpermanganat ist.

4. Verfahren nach den Ansprüche 1, 2 und 3, dadurch gekennzeichnet, dass man die Reaktion zwischen −10°C und 20°C ausführt.

5. Verfahren nach Anspruch 4, dadurch gekennzeichnet, dass man die Reaktion zwischen −10°C und 0°C ausführt.

**Revendications**

1. Procédé de préparation du 1,1-dioxyde d'acide 6-amino-pénicillanique, caractérisé en ce que l'acide 6-amino-pénicillanique ou le sulfoxyde d'acide 6-amino-pénicillanique est oxydé par un permanganate dans un milieu aqueux.

2. Procédé suivant la revendication 1, caractérisé en ce qu'un excès de permanganate est utilisé.

3. Procédé suivant les revendications 1 et 2, caractérisé en ce que le permanganate est le permanganate de potassium ou de sodium.

4. Procédé suivant les revendications 1, 2 et 3, caractérisé en ce que la réaction est exécutée entre −10°C et 20°C.

5. Procédé suivant la revendication 4, caractérisé en ce que la réaction est exécutée entre −10°C et 0°C.

5